(19) Europäisches Patentamt
European Patent Office
Office européen des brevets

(11) Numéro de publication: **0 088 024**
**B1**

(12) **FASCICULE DE BREVET EUROPEEN**

(45) Date de publication du fascicule du brevet: **17.09.86**

(21) Numéro de dépôt: **83400401.2**

(22) Date de dépôt: **25.02.83**

(51) Int. Cl.[4]: **C 07 D 491/14,** A 61 K 31/44, A 61 K 7/42 // (C07D491/14, 311:00, 307:00, 221:00)

(54) **Pyrido(3,4-c)-psoralènes, obtention, applications en cosmétologie et à titre de médicament, et compositions cosmétiques et pharmaceutiques les contenant.**

(30) Priorité: **25.02.82 FR 8203157**

(43) Date de publication de la demande: **07.09.83 Bulletin 83/36**

(45) Mention de la délivrance du brevet: **17.09.86 Bulletin 86/38**

(84) Etats contractants désignés: **AT BE CH DE GB IT LI LU NL SE**

(56) Documents cités: **FR - A - 2 405 067**

(73) Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Bisagni, Emile, 16, rue Bossuet, F-91400 Orsay (FR)**
Inventeur: **Dubertret, Louis, 31, avenue du Président Coty, F-75014 Paris (FR)**
Inventeur: **Moron, Jacqueline, 6 Hameau de l'Yvette Bat.E, F-91190 Gif-sur-Yvette (FR)**
Inventeur: **Averbeck, Dietrich, 2bis av.général de Gaulle, F-94240 L'Hay-les-Roses (FR)**
Inventeur: **Papadopoulo née Starbova, Dora, 16, rue Albert Camus, F-78530 Buc (FR)**
Inventeur: **Blais née Durot, Joselyne, 8, rue A. Neyts, F-92260 Fontenay-aux-Roses (FR)**
Inventeur: **Vigny, Paul, 8, rue Lacaze, F-75014 Paris (FR)**
Inventeur: **Schwencke née Magana, Maria, Niève 2,Rés. du Château de Courcelle, F-91190 Gif-sur-Yvette (FR)**
Inventeur: **Moustacchi, Ethel-Ester, 9, Clos désiré No 1, F-91120 Palaiseau (FR)**
Inventeur: **Nocentini, Silvano, 32, rue Ph. De Girard, F-75010 Paris (FR)**
Inventeur: **Zajdela, François, 48, rue Velpeau, F-92160 Antony (FR)**

(74) Mandataire: **Phélip, Bruno et al, c/o Cabinet Harlé & Phélip 21, rue de la Rochefoucauld, F-75009 Paris (FR)**

EP 0 088 024 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du psoralène. Elle est plus particulièrement relative à des dérivés mono-fonctionnels du psoralène consistant en des pyrido [3,4-c]psoralènes. L'invention a également pour objet un procédé d'obtention de tels composés. L'invention concerne aussi l'application desdits composés en cosmétologie, notamment pour stimuler la pigmentation de la peau, ainsi qu'à titre de médicaments, en particulier pour le traitement des affections cutanées. L'invention est aussi relative à des compositions cosmétiques et pharmaceutiques contenant, à titre d'agent actif, lesdits composés.

La photochimiothérapie, qui repose sur l'activation par les ultraviolets A, dans la peau humaine, de molécules photoactives de la famille des furocoumarines, les psoralènes, se développe rapidement en dermatologie. Il s'agit en effet d'une technique thérapeutique particulièrement efficace et commode dans le traitement des dermatoses inflammatoires chroniques bénignes, comme le psoriasis qui touche 2 à 3% de la population mondiale, et dans le traitement de dermatoses malignes, comme le mycosis fongoïde, lymphome cutané malin, rare mais d'évolution paritculièrement sévère. Cette technique thérapeutique est également employée avec un certain succès dans le traitement de nombreuses maladies inflammatoires chroniques de la peau, comme l'eczéma atypique, le lichen plan, le parapsoriasis en goutte, le prurit des hémodialysés, ainsi que dans les photodermatoses et dans les maladies dépigmentantes comme le vitiligo.

On a déjà proposé de préparer des dérivés du psoralène et de les utiliser pour le traitement des affections cutanées, particulièrement du psoriasis. A titre de référence bibliographique illustrant l'art antérieur dans ce domaine, on peut citer la demande de brevet FR 7 824 754 (publication 2 405 067) déposée le 25 août 1978 au nom de l'AGENCE NATIONALE DE VALORISATION DE LA RECHERCHE (ANVAR) ayant pour tire «Compositions pharmaceutiques contenant des dérivés mono-fonctionnels du psoralène pour le traitement des affections cutanées». Cette demande de brevet, à laquelle l'homme de l'art pourra se référer si besoin est, expose d'une manière détaillée les connaissances de l'époque relatives aux dérivés du psoralène et à leur application comme médicament.

D'une manière résumée, les psoralènes sont utilisés en association avec la lumière ultraviolette pour la photothérapie des maladies cutanées, telles que le psoriasis. Ce traitement est connu sous le nom de PUVA-thérapie. Des travaux récents ont cependant montré que certains psoralènes bi-fonctionnels, tels que le 8-méthoxypsoralène, connu sous l'abréviation 8-MOP, et la 5-méthoxypsoralène, en abréviation 5-MOP, sont cancérigènes chez la souris après exposition à de la lumière se situant dans le proche ultraviolet aux alentours de 365 nm. Au contraire, le 3-carbéthoxypsoralène, qui est un dérivé mono-fonctionnel du psoralène, est totalement inoffensif de ce point de vue, tout en conservant une activité thérapeutique vis-à-vis des malades atteints de psoriasis.

Les psoralènes actuellement utilisés en thérapie, tels le 8-méthoxypsoralène ou le 5-méthoxypsoralène, sont des agents capables de réaliser des pontages dans l'ADN, pontages biologiquement difficiles à réparer et capables d'introduire des erreurs dans la réplication génétique. Cette propriété explique sans doute leur haute activité mutagène et leur propriété carcinogène chez l'animal. Il s'agit donc de médicaments efficaces mais peutêtre non dénués de risques en utilisation chronique chez l'homme. C'est pourquoi, la photochimiothérapie est interdite chez les enfants jusqu'à l'âge de 18 à 20 ans et l'on essaye de l'utiliser le moins possible avant la cinquantaine.

En cosmétologie, on connaît aussi des produits qui stimulent la pigmentation et sont constitués de dérivés du psoralène, tels que le 5-méthoxypsoralène ou 5-MOP. Ils trouvent application dans des compositions cosmétiques pour le brunissage de la peau et/ou pour la protection de celle-ci contre le soleil. L'inconvénient de tels produits est d'être mutagènes.

L'invention a pour objet de nouveaux dérivés mono-fonctionnels du psoralène. Ces composés sont utiles à titre de médicaments, notamment dans le domaine du traitement des affections cutanées et plus spécialment pour le traitement des dermatoses inflammatoires bénignes et malignes, à savoir le psoriasis, le mycosis fongoïde, les eczémas constitutionnels et de contact, les parapsoriasis en plaques et en gouttes, les pelades, les prurigos, les lichens plans, les urticaires pigmentaires ainsi que les troubles de la pigmentation et les photodermatoses.

Les composés de l'invention qui possèdent l'aptitude de stimuler la pigmentation de la peau, sont également utiles dans le domaine cosmétique.

Les nouveaux composés de l'invention sont des pyrido[3,4-c]psoralènes ou pyrido[3,4-c] furo[3,2-g] coumarines répondant à la formule générale I

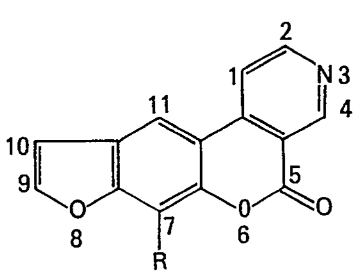

(I)

formule dans laquelle le radical R représente un atome d'hydrogène, un groupe alkyle inférieur en $C_1$-$C_4$, de préférence un radical méthyle ou un groupe alcoxy inférieur en $C_1$-$C_4$, de préférence le radical méthoxy.

Pour obtenir les composés de formula I, on fait

réagir un acétate d'hydroxy-6 R-7 dihydro-2,3 benzofuranne, où R est défini comme ci-dessus, sur une carbéthoxy-3 pipéridone-4, ce qui conduit à une hexahydro-1,2,3,4,9,10 R-7 pyrido[3,4-c]furo[3,2-g] coumarine, pouvant être substituée en position 3, et on soumet cette dernière à une réaction d'aromatisation au cours de laquelle on réalise à la fois la déshydrogénation et l'enlèvement d'un groupement substituant en 3.

Le procédé de l'invention sera davantage illustré dans la description qui suit.

A titre de produit de départ on utilise un composé de formule II

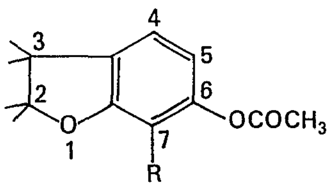

(II)

dans la laquelle R a la signification mentionnée précédemment. Un tel composé peut être obtenu conformément au procédé décrit par E.C. HORNING et al. dans J. Am. Chem. Soc. 70 (1948) page 3619 sous le titre «Furocoumarins. Synthesis of 2,3-dihydropsoralen». D'une manière résumée, ce procédé consiste à faire réagir sur la résorcine, portant en 2 le groupe R défini comme ci-dessus, du chloroacétonitrile en présence d'acid chlorhydrique et de chlorure de zinc, à traiter l'intermédiaire par de l'acétate de sodium ou de potassium, ce qui fournit une hydroxybenzofuranone, laquelle est ensuite acétylée par l'anhydride acétique puis réduite par voie catalytique, par exemple en présence de charbon palladié, pour donner respectivement l'acétate d'hydroxy-6 R-7 dihydro-2,3 benzofuranne de formule II où R est défini comme précédemment. L'autre produit de départ est une carbéthoxy-3 pipéridone-4 qui est disponible sur le marché. Pour le procédé de l'invention on peut utiliser la carbéthoxy-3 pipéridone-4 elle-même ou un dérivé substitué en position 1 par exemple par une groupe méthyle, ou de préférence, la benzyl-1 carbéthoxy-3 pipéridone-4 de formule IIIa.

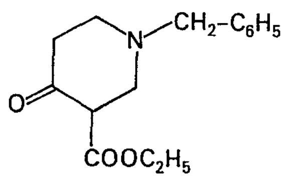

(IIIa)

La première étape du procédé de l'invention consiste à faire réagir le composé de formule II sur la carbéthoxy-3 pipéridone-4, en particulier celle représentée par la formule IIIa. Cette réaction est connue de l'homme de l'art sous le nom de réaction de VON PECHMANN (Oragnic Reactions T. 7, page 1).

Il s'agit d'une réaction de synthèse des coumarines consistant à condenser un béta-cétoester sur un phénol, en particulier un polyphénol. La réaction est réalisée en milieu acide. Dans le cas de la présente invention, on utilise avantageusement un milieu d'acide acétique contenant du gaz chlorhydrique. Le milieu acide préféré est constitué d'acide acétique contenant environ 6% en poids d'acide chlorhydrique gazeux sec. La réaction est réalisée de préférence à la température ordinaire. Il n'y a pas intérêt à augmenter la température car cela occasionne généralement une baisse des rendements. La réaction est donc poursuivie sous agitation pendant plusieurs jours. On obtient alors un rendement de l'ordre de 75% ou davantage. Cette première étape conduit à une hexahydro-1,2,3,4,9,10 R-7 pyrido[3,4-c]furo[3,2-g] coumarine pouvant être substituée en 3, de préférence par un groupe benzyle. Dans ce dernier cas le produit intermédiaire peut être représenté par la formule IVa

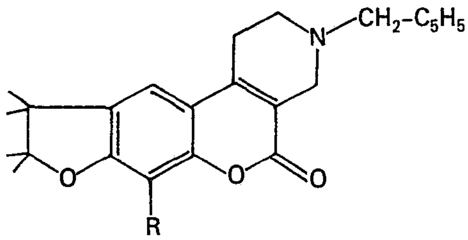

(IVa)

Dans les conditions précitées, on obtient directement le produit sous forme de chlorhydrate et il n'est par nécessaire d'isoler la base correspondante. Au contraire, le procédé permet de traiter directement le chlorhydrate avec un rendement amélioré.

La deuxième étape du procédé de l'invention consiste en une réaction d'aromatisation au cours de laquelle il y a à la fois déshydrogénation et enlèvement d'un groupement substituant en 3. La réaction peut être réalisée dans un solvant en présence d'un agent de déshydrogénation ayant une forte activité, de préférence le palladium sur charbon, par exemple à 10%. Le solvant doit être capable de travailler au reflux tout en restant inerte vis-à-vis des réactifs en présence. Les solvants qui ont donné de bons résultats sont le diphényléther ou la décaline.

On obtient ainsi les pyrido[3,4-c]psoralènes de formule I.

L'homme de l'art trouvera dans les exemples 1 à 4 ci-après les détails sur les modes opératoires permettant de préparer les nouveaux dérivés du psoralène de formule I.

Les nouveaux composés de formule I sont des médicaments utiles pour le traitement des affections cutanées et plus spécialement pour le traitement des dermatoses inflammatoires bénignes et malignes, à savoir le psoriasis, le mycosis fongoïde, les eczémas constitutionnels et de contact, les parapsoriasis en plaques et en gouttes, les pelades, les prurigos, les lichens plans, les urticaires pigmentaires ainsi que les troubles de la pigmentation et les photodermatoses.

Ils possèdent des propriétés photobiologiques

très favorables dans le traitement par PUVA-thérapie des maladies de la peau. Le traitement des affections cutanées avec les composés selon la présente invention consiste à administrer par voie orale ou topique une quantité efficace d'un composé de formule I et à soumettre le patient à de la lumière dans le proche UV.

Il a été constaté à présent que, pour l'application topique, des pommades ou des solutions contenant environ 0,1% à environ 2% (en poids) du composé thérapeutiquement efficace peuvent être utilisées. Des concentrations d'environ 0,5% en poids sont préférées.

Comme excipients pour les solutions ou les pommades selon l'invention, on peut utiliser tous les excipients couramment employés qui sont bien connus de l'homme de l'art. Des exemples de tels excipients sont cités par Schaefer et al. dans Archiv. of Dermatology (1976).

Il es bient entendu possible également d'incorporer les composés de la présente invention dans d'autres compositions, solutions ou pommades ou d'ajouter des parfums, des colorants, des filtres solaires ou des agents de conservation, ainsi que tous autres composés couramment utilisés. Il est également possible de combiner deux ou plusieurs agents photosensibilisateurs ou photoprotecteurs.

Pour l'administration orale, on préfère en particulier des quantités comprises entre 0,5 et 2 mg/kg.

Ainsi qu'il est mentionné plus haut, le traitement des affections cutanées avec les composés selon l'invention comprend l'irradiation par de la lumière dans le proche ultraviolet (UVA) avec un spectre comprenant essentiellement des longueurs d'onde comrpises entre 320 et 380 nm. Ainsi qu'on l'expliquera plus loin, la dose d'irradiation à chaque séance peut être d'emblée de 5 $J/cm^2$ 10 $J/cm^2$. Néanmoins, des doses allant jusqu'à 20 $J/cm^2$ peuvent être utilisées pendant une courte période de temps.

Les composés de l'invention trouvent également application en cosmétologie, en raison de leur propriété de stimuler la pigmentation de la peau. L'invention a donc pour objet des compositions cosmétiques contenant une quantité d'au moins un composé selon l'invention efficace pour réaliser la pigmentation de la peau, en association avec un véhicule convenant à l'application externe. Les véhicules de telles compositions sont bien connus de l'homme de l'art et n'ont pas besoin d'être décrits plus en détails. Pour les composés de l'invention on peut utiliser des véhicules semblables à ceux déjà proposés pour les compositions cosmétiques contenant du 5-MOP. Les compositions cosmétiques de l'invention se présentent sous forme de crème, lait, huile, aérosols (sprays) et tous autres produits à usage externe. Les composés de l'invention peuvent, au sein de telles compositions, être associés ou non à des filtres ou écrans solaires. La quantité de composé actif à mettre en œuvre dans les compositions cosmétiques de l'invention n'est pas critique et peut varier selon la destination de celles-ci. En général des quantités de 0,01 à 0,5% en poids par rapport à la composition sont convenables.

Les nouveaux composés de l'invention présentent l'ensemble des propriétés suivantes:

- une photoréactivité appréciable avec les acides nucléiques, produisant un effet antiprolifératif;
- l'absence d'induction de pontages dans l'ADN;
- une induction dans l'ADN de mono-additions uniquement, ayant pour conséquence l'arrêt des synthèses d'ADN et d'ARN;
- de faibles effets phototoxiques, donc provoquant peu ou pas d'érythème;
- un faible pouvoir mutagène, ce qui contraste avec les furocoumarines bi-fonctionnelles actuellement employées en PUVA-thérapie, qui portent un risque cancérigène appréciable.

On donnera maintenant des exemples de préparation des composés de l'invention.

Exemple 1

Etape (a): Hexahydro-1,2,3,4,9,10 benzyl-3 pyrido[3,4-c]furo[3,2-a]coumarine (formule IVa, R = H)

Un mélange du composé II avec R = H (2,5 g, 14 mmoles) et du chlorhydrate de benzyl-1-carbéthoxy-3-pipéridone-4 (formule IIIa) (4g, 13,5 mmoles) en solution dans 25 ml d'acide acétique glacial contenant 6% d'acide chlorhydrique sec est agité à température ambiante pendant 5 jours. Le précipité formé est essoré, lavé à l'acide acétique, et séché. On obtient 3,78 g (76%) du chlorhydrate (IVa) avec R = H, F = 234–236°C.

310 mg de ce chlorhydrate en suspension dans 20 ml d'eau sont neutralisés par une solution saturée d'hydrogénocarbonate de sodium. Après 0,50 heure d'agitation, le précipité est essoré, lavé à l'eau, séché et recristallisé dans l'éthanol pour donner 206 mg de cristaux, F = 163–165°C.

RMN ($CDCl_3$, δppm, 60 MHz) 2,73 (4H, s large, $CH_2$ en 1 et 2); 3,15 (2H, t, J = 8,5 Hz, $CH_2$ en 10); 3,4 (2H, s large, $CH_2$ en 4); 3,65 (2H, s, $CH_2$-$C_6H_5$); 4,56 (2H, t, J = 8,5 Hz, $CH_2$ en 9); 6,53 (1H, s, $H_7$); 7,16 (6H, s large, $C_6H_5$-$CH_2$ et $H_{11}$).

Analyse % calculé pour $C_{21}H_{19}NO_3$:

| | | | |
|---|---|---|---|
| | C 75,65; | H 5,74; | N 4,20 |
| Trouvé: | C 75,29; | H 5,65; | N 4,16 |

Etape (b): Pyrido[3,4-c]furo[3,2-g]coumarine (formule I, R = H, composé 1a)

2,33 g (6,31 mmoles) du chlorhydrate obtenu à l'étape (a) en suspension dans 30 ml de diphényléther sont chauffés à reflux pendant 5 heures en présence de 2,1 g de Pd/C à 10%. (Pd/C = charbon palladié).

Le charbon palladié est filtré à chaud, lavé avec 5 ml de diphényléther chaud. Après refroidissement on ajoute 500 ml d'hexane. Le précipité formé (573 mg) est essoré, lavé à l'hexane et séché.

Après deux cristallisations d'un mélange chlorure de méthylène/alcool on obtient 375 mg (25%) d'aiguilles incolores, F = 284–288°C.

Le lavage du charbon palladié avec du chlorure de méthylène et de l'alcool chaud fournit encore 95 mg de produit brut.

RMN ($CDCl_3$, δ ppm, 100 Hz) 6,90 (1H, dxd, J = 2,3 Hz, J = 0,2 Hz, $H_{10}$); 7,54 (1H, d, J = 1 Hz, $H_7$); 7,73 (1H, d, J = 2,3 Hz, $H_9$); 7,95 (1H, d, J = 5,5 Hz, $H_1$); 8,30 (1H, d, J = 0,4 Hz, $H_{11}$); 8,95 (1H, d, J = 5, 5Hz, $H_2$); 9,75 (H, s, $H_4$).

Analyse % calculé pour $C_{14}H_{17}NO_3$:

| | | | |
|---|---|---|---|
| | C 70,89; | H 2,97; | N 5,91 |
| Trouvé: | C 70,79; | H 3,06; | N 5,91 |

Exemple 2

Etape (a): Méthyl-7 hexahydro-1,2,3,4,9,10 benzyl-3-pyrido[3,4-c]furo[3,2-g]coumarine (formule IVa, R = $CH_3$)

Ce composé est préparé selon la mode opératoire décrit ci-dessus à l'exemple 1, étape (a). A partir de 3,90 G (20 mmoles) du composé (II) avec R = $CH_3$; et de 5,9 g (20 mmoles) de la pipéridone (IIIa) on obtient 5,85 g (15 mmoles, 75%) du chlorhydrate (IVa) avec R = $CH_3$, F = 260°C.

La base libérée est cristallisée dans l'éthanol, F = 208°C RMN ($CDCl_3$, δ ppm, 60 MHz) 2,25 (3H, s, $CH_3$ en 7); 2,77 (4H, s large, $CH_2$ en 1 et 2); 3,23 (2H, t, J = 8,5 Hz, $CH_2$ en 10); 3,5 (2H, s large, $CH_2$ en 4); 3,70 (2H, s, $CH_2$-$C_6H_5$); 4,66 (2H, t, J = 8,5 Hz, $CH_2$ en 9); 7,16 (1H, s, $H_{11}$); 7,35 (5H, s, $C_6H_5$-$CH_2$).

Analyse % calculé pour $C_{22}H_2NO_3$:

| | | | |
|---|---|---|---|
| | C 76,06; | H 6,05; | N 4,03 |
| Trouvé: | C 75,92; | H 6,12; | N 4,22 |

Exemple 3

Méthyl-7 pyrido[3,4-c]furo[3,2-g]coumarine (formule I, R = $CH_3$, composé 1b)

Le pyridopsoralène du titre est préparé comme décrit ci-dessus à l'exemple 1, étape (b). A partir de 3 g (7,83 mmoles) du chlorhydrate préparé à l'étape (a) de cet exemple, on obtient 928 mg de produit brut qui, après deux cristallisations du mélange $CH_2Cl_2/C_2H_5$ OH fournissent (487 mg (25%) de base, F = 272–274°C.

RMN ($CDCl_3$, δ ppm, 100 MHz) 2,65 (3H, s, $CH_3$ en 7), 6,87 (1H, d, J = 2,2 Hz, $H_{10}$); 7,74 (1H, d, J = 2,2 Hz, $H_9$); 7,93 (1H, d, J = 5,5 Hz, $H_1$); 8,15 (1H, s, $H_{11}$); 8,92 (1H, d, J = 5,5 Hz, $H_2$); 9,56 (1H, s, $H_4$).

Analyse % calculé pour $C_{15}H_9NO_3$:

| | | | |
|---|---|---|---|
| | C 71,71; | H 3,61; | N 5,57 |
| Trouvé: | C 71,58; | H 3,69; | N 5,63 |

Les nouveaux composés de l'invention ont été soumis à des essais permettant d'apprécier leur activité en cosmétologie et à titre de médicament.

La structure mono-fonctionnelle des composés, qui constitue une propriété importante vis-à-vis de leur utilisation thérapeutique et cosmétique, a été vérifiée par deux séries d'expériences photochimiques et photobiologiques.

Les composés 1a et 1b possèdent des propriétés spectroscopiques voisines, lesquelles diffèrent notablement de celles du psoralène. La première transition d'absorption ($\lambda_{max}$ = 330 nm) présente notamment un coefficient d'absorption moléculaire plus élevé à 365 nm.

Le composé 1a libre en solution, possède une bonne stabilité photochimique sous irradiation ultraviolette jusqu'à une dose incidente de 27 kJ/$m^2$. Des preuves d'une complexation importante du composé la avec l'ADN ont été obtenues par spectroscopie d'absorption et d'émission. La solubilité de ce composé dans l'ADN est nettement supérieure à celle du même composé dans un mélange alcool-eau (facteur au minimum supérieur à 10).

Des expériences de dénaturation-renaturation thermique effectuées sur de l'ADN modifié par le composé la en présence d'UV-A (dose incidente allant jusqu'à 27 kJ/$m^2$) conduisent à des valeurs de fraction non renaturante de 100%. Il est donc clair que se composé ne peut pas induire de biadditions sur l'ADN.

1) Utilisation de mutants de levure bloqués spécifiquement dans la réparation des pontages interbrins de l'ADN:

On sait que la photoaddition de furocoumarines bi-fonctionnelles induit à la fois des pontages interbrins et des mono-additions sur les bases de l'ADN. Celle de furocoumarines mono-fonctionnelles produit uniquement cette dernière réaction.

Pour les expériences, on a utilisé un mutant de levure, pso2, qui a les propriétés suivantes: a) Il est beaucoup plus sensible que le type sauvage dont il dérive à l'effet létal de la photo-addition des psoralènes bifonctionnels (Isolation and characterization of pso mutants sensitive to photo-addition of psoralen derivatives in Saccharomyces cerevisiae. J.A.P. HENRIQUES and E. MOUSTACCHI. Genetics 95, 273-288 (1980)), ainsi qu'à d'autres agents de pontage, tels que les moutardes azotées bi-fonctionnelles (Mutagenesis induced by mono- and bifunctional alkylating agents in yeast mutants sensitive to photoaddition of furocoumarins (pso). O. CASSIER et E. MOUSTACCHI. Mutation Res. 84, 37-47 (1981)), ou la mitomycine C. b) Il est relativement très peu sensible par rapport autype sauvage à la photo-addition de psoralènes mono-fonctionnelles. De même pso2 présente la même sensibilité que le type sauvage aux ultraviolets de 254 nm ou aux radiations ionisantes qui sont connus pour produire essentiellement des lésions des brins et des ruptures de l'ADN sans production de pontages aux doses biologiquement significatives.

c) On a démontré biochimiquement que le mutant pso2 est bloqué dans la réparation des pontages interbrins de l'ADN, (The fate of 8-methoxypsoralen photo-induced cross-links in nuclear and mitochondrial yeast DNA. Comparison of wild type and repair-deficient strains. N. MAGANA-SCHWENCKE, J.A.P. HENRIQUES, R. CHANET and E. MOUSTACCHI Proc. Natl. Acad. Sci. (USA), (1981)). Ce blocage est spécifique car le mutant pso2 répare comme le type sauvage les mono-additions photo-induites sur l'ADN.

Autrement dit, si le mutant pso2 se montre plus sensible que le type sauvage à l'effet létal d'un agent, on peut en déduire que cet agent est capable de produire des pontages interbrins de l'ADN, de sorte qu'il s'agit d'un composé bi-fonctionnel. En revanche, si le mutant pso2 a la même sensiblité que le type sauvage à un agent donné, on en conclut que cet agent ne provoque pas de pontages et qu'il est par conséquent de type monofonctionnel.

On constate que le mutant pso2 a la même sensibilité que le type sauvage à la photo-addition des produits 1a et 1b. Le mutant ayant la même capacité que le type sauvage à réparer les lésions de mono-addition, il est clair que ces nouveaux produits sont de type mono-fonctionnel. On confirme par ailleurs sur les souches de levure que ces nouveaux produits sont très photo-réactifs.

2) Vérification biochimique directe de l'absence de pontage dans l'ADN de cellules traitées par les produits 1a, 1b plus une irradiation à 365 nm.

La validité des conclusions précédentes est confirmée par l'analyse biochimique in vivo. En effet, l'ADN de cellules de type sauvage a été extrait immédiatement après le traitement avec les produits 1a, 1b à concentration $10^{-5}$M plus une irradiation par deux doses de radiations de 365 nm (30% et 5% de survie). Après séparation de l'ADN nucléaire de l'ADN mitochondrial en gradient de densité de chlorure de cesium, l'ADN est cassé de manière à avoir des segments de taille homogène (1 pontage en moyenne par molécule pour le 8-méthoxypsoralène), il est dénaturé puis renaturé. Si l'ADN contient des ponts interbrins, il se renature et se retrouve sous la forme double chaîne séparable en gradient de densité de l'ADN simple chaîne. Par contre quand l'ADN ne contient pas de pontage, il reste sous forme simple chaîne après renaturation. C'est cette dernière situation que l'on observe expérimentalement avec les produits 1a, 1b. Les expériences de contrôle avec le 8-méthoxypsoralène effectuées dans les mêmes conditions mettent en évidence de l'ADN ponté en double brin. Pour les détails de la technique utilisée voir: The fate of 8-methoxypsoralen photo-induced cross-links in nuclear and mitochondrial yeast DNA. Comparison of wild type and repair-deficient strains (N. MAGANA-SCHWENCKE, J.A.P. HENRIQUES, R. CHANET and E. MOUSTACCHI. Proc. Natl. Acad. Sci. (USA), (1981) et absence de pontages interchaînes dans l'ADN traité par le 3-carbéthoxypsoralène et une irradiation à 365 nm (N. MAGANA-SCHWENCKE, D. AVERBECK, J.A.P. HENRIQUES et E. MOUSTACCHI. C.R. Acad. Sci. Paris 291, 207–210 (1980)).

En résumé l'utilisation du mutant pso2 et l'examen direct de l'ADN de cellules traitées in vivo montrent clairement que les produits 1a, 1b sont bien de type mono-fonctionnel.

Pour la détection de l'activité photobiologique, le système eukaryote unicellulaire de la levure Saccharomyces cerevisiae s'est révélé très utile. Cette activité photobiologique a en effet été définie par l'induction d'effets létaux, l'induction de mutations cytoplasmiques «petite colonie» (dommages dans l'ADN mitochondrial) et l'induction de mutations nucléaires (réverses et aller). Les expériences ont été faites selon les méthodes habituellement utilisées et conformément aux références bibliographiques ci-après:

AVERBECK, D., BISAGNI, E., MOUSTACCHI, E. (1978) Biochim. Biophys. Acta 518, 464.

AVERBECK, D., MOUSTACCHI, E. (1979) Mutation Res. 88, 133.

AVERBECK, D., MOUSTACCHI, E. (1980) Photochem. Photobiol. 31, 475.

AVERBECK, D., AVERBECK, S., DALL'ACQUA, F. (1981) IL FARMACO 36, 492.

Dans les essais sur l'induction des effets létaux chez la levure (c'est-à-dire sur l'induction de l'inhibition de la capacité des cellules de former une colonie) on a observé qu'en présence de radiation à 365 nm (UVA) et à des concentrations équimolaires ($5.10^{-5}$M) le composé 1a, (exemple 1) montre une activité voisine de celle du 8-méthoxypsoralène (8-MOP), agent bifonctionnel largement utilisé en PUVA-thérapie.

Les courbes de survie établies à une concentration de $5.10^{-6}$M ont montré que les composés 1a (exemple 1) et 1b (exemple 2) sont respectivement 2,5 et 5 fois plus actifs sur la survie cellulaire que le 8-MOP. Ainsi, la dose d'UVA doit être 2,5 et 5 fois plus faible dans le cas des composés 1a et 1b que dans le cas du 8-MOP pour atteindre la même survie. A des concentrations équimolaires ($5.10^{-6}$M) le composé 1b a montré une activité voisine de celle du 4,5',8-triméthylpsoralène (4,5',8-TMeP) utilisé en photochimiothérapie du vitiligo et du psoriasis (voir PATHAK, M.A., PARRISH, J.A., FITZPATRICK, T.B. (1981) Il Farmaco Ed. Sc. 36,479-491). D'ailleurs la solubilité dans l'eau des composés 1a et 1b est semblable à celle du 4,5', 8-triméthylpsoralène.

Il est important de signaler que les deux composés 1a et 1b n'exercent pas chez la levure d'activité photodynamique due à la présence d'oxygène sur l'induction des dommages létaux. Ils sont en cela comparables aux furocoumarines comme le 8-MOP et le 4,5'-diméthylangélicine (4,5'-DMA) mais différents du 3-carbéthoxypsoralène (3-CPs). La photoaffinité des composés 1a et 1b pour l'ADN in vivo est environ, respectivement, 10 et 20 fois plus forte que celle du 8-MOP, donc nettement plus élevée que celle de la furocoumarine monofonctionnelle 3-CPs.

Si l'on considère l'induction de mutations cytoplasmiques en fonction des survivants, les composés 1a, 1b, dérivés monofonctionnels du psoralène, montrent à 10% de survivants un taux d'induction de mutations cytoplasmiques supérieur à 50%.

L'induction de mutations nucléaires (réversions et mutations «aller») a été déterminée selon le protocole décrit dans les articles susmentionnés. En présence de composés 1a ou 1b à une concentration de $5.10^{-6}M$ les réversions $His^+$ dans la souche haploïde de la levure Saccharomyces cerevisiae sont induites à une fréquence inférieure à celle observée après traitement avec le 3-CPs, donc à une fréquence très inférieure à celle observée après traitement avec les agents bifonctionnels 8-MOP et 4,5', 8-TMeP au même taux de survivants.

L'analyse du pouvoir d'induction de mutations aller, c'est-à-dire de mutations vers la résistance à la canavanine aboutit à la même conclusion: en fonction des doses d'UVA, on obtient avec les composés 1a et 1b $5.10^{-6}M$ des résultats comparables à ceux obtenus avec le 3-CPs à $5.10^{-5}M$. En fonction des fractions de survivants, les composés 1a et 1b sont nettement moins mutagènes que le 3-CPs et les composés bifonctionnels 8-MOP et 4,5', 8-TMeP.

L'effet thérapeutique des nouvelles furocoumarines a été encore apprécié d'après leur capacité à inhiber les synthèses cellulaires comme l'avait déjà montré BORDIN et al en 1972 avec d'autres furocoumarines. (Bordin, F., Baccichetti, F. and Musajo, L. (1972) Experientia 28, 148).

On a mesuré dans deux lignées fibroblastiques humaines en culture (GM 1603 et Ja...) l'effet photosensibilisateur des composés 1a et 1b comparativement à celui du 8-méthoxypsoralène (8-MOP) et du 3-carbéthoxypsoralène (3-CPs) sur la synthèse de l'ADN, de l'ARN et des protéines.

Les cellules cultivées en boîtes de Petri en milieu minimum de Eagle, additionné de 10% de sérum embryonnaire de veau, ont été marquées par des précurseurs spécifiques en phase exponentielle de croissance. Les taux des synthèses ont été estimés en mesurant l'incorporation des précurseurs radioactifs dans du matériel acido-précipitable (voir Nocentini S. (1978) Biochim. Biophys. Acta 521, 160).

Les résultats indiquent que la photosensibilisation de fibroblastes humains par ces différentes furocoumarines diminue la synthèse de l'ADN et de l'ARN en fonction de la dose d'UV-A (365 nm). Les composés 1a et 1b ont une efficacité d'inhibition du même ordre. Ils sont légèrement plus puissants que le 8-MOP et beaucoup plus que le 3-CPs dans les conditions expérimentales utilisées. Pour tous les produits, l'inhibition de la synthèse de l'ARN est légèrement plus faible que celle de l'ADN à une dose d'irradiation identique. La synthèse des protéines est par contre peu affectée.

En conclusion, les résultats ci-dessus indiquent que les composés 1a et 1b ont une photoaffinité, vis-à-vis de l'ADN nettement plus grande que celle du 8-MOP et une photoréactivité qui avoisine celle du 4,5',8-triméthylpsoralène. Les résultats sur l'induction de mutations cytoplasmiques «petites» (dommages dans l'ADN mitochondrial) et sur l'induction de mutations nucléaires montrent que les deux composés photoréagissent de façon monofonctionnelle avec l'ADN, c'est-à-dire qu'ils sont incapables de former des pontages dans l'ADN.

Les résultats ci-dessus ont encore été confirmés par des essais destinés à mesurer sur des cultures cellulaires l'effet antiprolifératif des nouveaux composés de l'invention.

L'activité photobiologique des deux composés monofonctionnels 1a et 1b a été étudiée dans des cellules de mammifères in vitro, afin de déterminer leur effet anti-prolifératif. Cet effet a été comparé avec celui du 8-méthoxypsoralène (8-MOP), furocoumarine bifonctionnelle.

Une lignée diploïde de cellules de hamster chinois V79 a été utilisée.

On a déterminé l'action des composés sur la survie cellulaire (effet antiprolifératif) en mesurant leur pouvoir d'inhiber la capacité des cellules à former des colonies. La technique utilisée est celle décrite par C.F. Arlett (1977). (C.F. ARLETT. «Mutagenicity testing with V79 chinese hamster cells». In Handbook of Mutagenicity Test Procedure, edited by B.J. Kilbey, Elsevier/North Holland Biomedical Press, 1977, p. 175–192).

L'effet antiprolifératif des composés 1a et 1b a été déterminé pour une concentration de $5.10^{-6}M$. Les doses de rayonnement UVA de 365 nm s'échelonnaient entre 200 et 3200 $J.m^{-2}$. En absence de rayonnement, on n'a observé aucune diminution de la survie cellulaire pour chacun des deux composés.

Dans le but de comparer les effets antiprolifératifs des composés étudiés, on a déterminé les doses $LD_{10}$ qui laissent 10% de survivants. Ces valeurs $LD_{10}$ sont respectivement de 540 et 1800 $J.m^{-2}$ pour 1b et 1a. Le composé 1b est donc nettement plus efficace que le composé 1a, les deux représentant un effet de photosensibilisation considérable.

En ce qui concerne la comparaison avec la furocoumarine bifonctionnelle, 8-MOP, largement utilisée actuellement en PUVA-thérapie, on peut noter que, pour la même concentration ($5.10^{-6}M$), la dose $LD_{10}$ est 3800 $J.m^{-2}$. Ceci permet d'affirmer que les deux nouveaux composés 1a et 1b, tout en étant monofonctionnels, ont comparativement au 8-MOP, une action antiproliférante plus forte.

En comparaison avec les furocoumarines actuellement utilisées en photochimiothérapie (PUVA) à savoir le 8-MOP et le 4,5',8-TMeP la capacité antiproliférative des nouveaux produits 1a et 1b est comparable à celle des furocoumarines bifonctionnelles tout en comportant moins de risques mutagènes et cancérigènes. Ces nouveaux composés possèdent donc une activité photothérapeutique plus intéressante que celle des composés analogues connus et utilisés.

L'activité clinique et thérapeutique des composés 1a et 1b a été encore mesurée sur la peau humaine.

On a dilué 10 mg des composés 1a et 1b dans 2

g d'hydrocérine ROC (lanoline purifiée) rendue liquide par un chauffage à 60°C.

Dans un premier temps cette préparation topique a été testée sur la peau humaine normale (avant-bras). Une quantité de 10 microgrammes/$cm^2$ des produits 1a et 1b a été appliquée sur une surface cutanée circulaire de 3 cm de diamètre. Les deux produits ont été laissés sur la peau pendant 2 heures, afin d'assurer une pénétration cutanée suffisante. Après ces 2 heures, une irradiation aux ultraviolets A à 5 J/$cm^2$ a été appliquée sur les deux zones testées et aucun érythème précoce ni retardé n'a été observé avec ces doses d'irradiation. Dans un second temps, la même expérience a été répétée mais avec une irradiation de 20 J/$cm^2$ pratiquée à travers une plaque de verre afin de supprimer tout effet des ultraviolets B. Dans ces conditions, un léger érythème est apparu, 72 heures après l'irradiation. Cet érythème, à la limite du visible, a persisté 18 jours. Les photographies ont été prises au 6ème jour mais également une étude photopléthysmographique par réflexion a pu être réalisée et a permis d'objectiver un doublement de l'impulsion systolique des capillaires au niveau des zones érythémateuses. L'érythème ainsi que les résultats de la photopléthysmographie par réflexion ont été exactement identiques avec les produits 1a et 1b. Il n'y a eu, en particulier, aucune desquamation ni aucune douleur locale au cours de l'évolution de ces érythèmes. On peut conclurer de ces tests que les produits 1a et 1b sont très discrètement érythématogènes dans des conditions où le 3-carbéthoxypsoralène ne l'est pas. L'érythème n'aparaît que pour des doses d'irradiation qui sont le double de celles habituellement utilisées pour une séance en thérapeutique.

Les produits 1a et 1b ont été également testés dans le même excipient sur une plaque de psoriasis très inflammatoire. Ces deux produits ont été appliqués sur des surfaces de 3 cm de diamètre. Sur une troisième surface, l'excipient seul a été appliqué. Les doses de produit 1a et 1b appliquées ont été de 30 microgrammes/$cm^2$. L'application a été faite, ainsi que celle de la lanoline seule, 2 heures avant les séances d'irradiation. Les doses d'irradiation ont été d'emblée de 5 J/$cm^2$ et les séances se sont répétées tous les deux jours, soit plus précisément chaque lundi, mercredi et vendredi matin. Dès la 4ème séance on observait une discrète diminution des squames au niveau des zones où étaient appliqués les produits 1a et 1b ainsi qu'une diminution palpable de l'infiltration. A cette date, les photographies ne permettaient pas d'objectiver de différence. Le blanchiment complet des zones traitées par les produits 1a et 1b a été obtenu à la 10ème séance, soit après une dose totale d'irradiation de 50 J/$cm^2$. Ce blanchiment a été pratiquement aussi rapide sur les zones traitées par les produits 1a et 1b. La zone traitée par la lanoline est restée absolument sans modification. Au cours de ce traitement aucun érythème ni effet secondaire n'ont été observés au niveau des zones traitées par les produits 1a et 1b cependant on observe une hyperpigmentation au niveau de ces deux zones en particulier pour 1b.

Ce test thérapeutique, comportant l'application deux heures avant les irradiations UVA de 30 µg par $cm^2$ des produits 1a et 1b, a été pratiqué sur 3 patients. Dans deux cas le produit 1b s'est montré discrètement supérieur au produit 1a et en particulier plus pigmentogène. Dans le troisième cas, seul le produit 1b a permis un blanchiment complet du psoriasis, le produit 1a n'ayant pas eu d'action antipsoriasique décelable. Il semble donc que le produit 1b ait une action antipsoriasique supérieure à celle du produit 1a après un traitement particulièrement court puisque se limitant à 10 séances de PUVA, soit une dose cumulative de 50 J/$cm^2$.

Un second test thérapeutique, destiné à comparer l'efficacité antipsoriasique du 8-méthoxypsoralène et du produit 1b a été entrepris chez 3 patients. Les concentrations de 8-MOP et du produit 1b ont été de $10^{-2}$M dans le même excipient que celui utilisé dans le test thérapeutique précédent. Ces deux préparations ont été appliquées sur deux zones arrondies de 3 cm de diamètre, situées sur la même plaque de psoriasis et ont été irradiées avec le même protocole de montée progressive en ultra-violet A, jusqu'à une dose maximum par séance de 10 J/$cm^2$. Pour ces trois malades, le produit 1b s'est montré plus efficace que le 8-méthoxypsoralène. Un malade est resté 15 jours après la fin du test sans aucun traitement et l'on a pu noter qu'il se produisait une rechute rapide au niveau de la zone traitée par le 8-méthoxypsoralène, alors que la zone correspondante traitée par le produit 1b ne rechutait pratiquement pas dans ce délai. Ce test thérapeutique complémentaire permet donc de montrer que le produit 1b est plus efficace que le 8-méthoxypsoralène et, qu'après traitement, les rechutes se font moins rapidement que le produit 1b qu'avec le 8-méthoxypsoralène.

Les propriétés pigmentogènes des produits 1a et 1b ont été testées sur la peau normale à la concentration de 0,5%. Une pigmentation visible a été observée chez les 3 sujets normaux testés avec le produit 1b dans trois cas sur trois après des doses cumulatives d'UVA de 140 J/$cm^2$. Par contre, avec le produit 1a une pigmentation très légère, à peine visible, n'a été observée que dans deux cas sur trois. Il semble que les propriétés pigmentogènes du produit 1b soient donc supérieures à celle du produit 1a.

En conclusion, les composés 1a et 1b ont une bonne activité antipsoriasique en utilisation par voie topique. Le produit 1b se révèle plus efficace que le 8-méthoxypsoralène employé dans des conditions de concentration et d'irradiation strictement identiques. Les composés 1a et 1b sont pigmentogènes et le produit 1b est plus pigmentogène que le produit 1a. Enfin, les produits 1a et 1b ne sont pas érythematogènes dans les conditions d'irradiation habituellement utilisées en thérapeutique humaine. Les produits 1a et 1b

sont par ailleurs, comme il a été montré, moins mutagènes que le 3-carbéthoxypsoralène.

Les meilleurs résultats thérapeutiques ont été obtenus jusqu'à présent avec le composé 1b. Ces nouveaux composés, 1a et 1b, peuvent donc être utilement mis en œuvre dans la photochimiothérapie de tous les psoriasiques, même dans la photochimiothérapie de l'enfant. Cette possibilité de traiter les enfants est particulièrement intéressante car il n'existe actuellement que peu de thérapeutique permettant de les traiter de façon efficace.

En raison de leurs effets pigmentogènes, les nouveaux composés 1a et 1b peuvent trouver également une application en cosmétique.

**Revendiactions pour les états contractants: BE, CH, DE, GB, IT, LI, LU, NL, SE**

1. Pyrido[3,4-c]psoralènes ou pyrido[3,4-c] furo[3,2-g]coumarines répondant à la formule générale I:

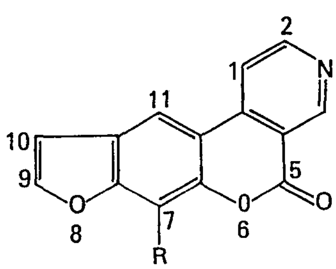

(I)

formule dans laquelle R représente un atome d'hydrogène, un groupe alkyle inférieur en $C_1$-$C_4$ ou un groupe alkoxy inférieur en $C_1$-$C_4$.

2. Composé selon la revendication 1 répondant à la formule I dans laquelle R est un groupe méthyle.

3. Composé selon la revendication 1 répondant à la formule I dans laquelle R est un groupe méthoxy.

4. Composé selon la revendication 1 répondant à la formule I dans laquelle R est un atome d'hydrogène.

5. Procédé pour l'obtention des pyrido [3,4-c]furo[3,2-g]coumarines de formule I, carctérisé en ce que l'on fait réagir un acétate d'hydroxy-6 R-7 dihydro-2,3 benzofuranne de formule II:

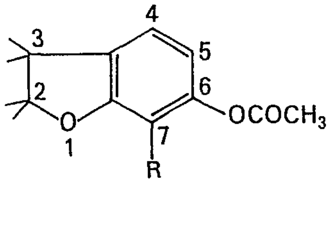

(II)

dans laquelle R est comme défini à la revendication 1, avec une carbéthoxy-3 pipéridone-4 de formule III:

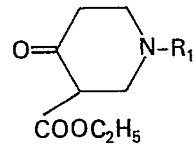

(III)

dans laquelle $R_1$ représente un atome d'hydrogène on un substituant, ce qui conduit à une hexahydro-1,2,3,4,9,10 $R_1$-3 R-7 pyrido[3,4-c]furo [3,2-g]coumarine de formule IV:

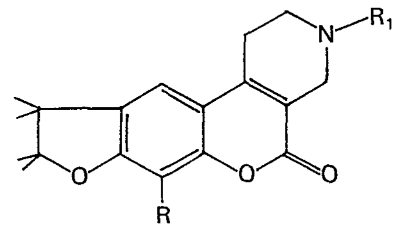

(IV)

dans laquelle R et $R_1$ sont définis comme précédemment, et que l'on soumet cette dernière à une réaction d'aromatisation au cours de laquelle on réalise à la fois la déshydrogénation et l'enlèvement d'un substituant $R_1$.

6. Procédé selon la revendication 5, caractérisé en ce que $R_1$ est un groupe benzyle.

7. Procédé selon l'une des revendications 5 ou 6, caractérisé en ce que la première étape est réalisée en milieu acide.

8. Procédé selon la revendication 7, caractérisé en ce qu'on travaille en présence d'acide acétique contenant du gaz chlorhydrique.

9. Procédé selon l'une quelconque des revendications 5 à 8, caractérisé en ce que la réaction d'aromatisation est effectuée dans un solvant choisi parmi le diphényléther et la décaline, en présence d'un catalyseur de déshydrogénation.

10. Procédé selon la revendication 9, caractérisé en ce que le catalyseur de déshydrogénation est du palladium sur charbon.

11. Composition pharmaceutique contenant à titre d'agent actif au moins l'une des pyrido[3,4-c]furo[3,2-g]coumarines définies dans les revendications 1 à 4 en association avec un véhicule pharmaceutiquement acceptable.

12. Composition pharmaceutique selon la revendication 11 pour administration par voie orale, caractérisée en ce qu'elle comprend une quantité suffisante pour que la dose unitaire administrée soit 0,5 à 2 mg/kg de poids corporel.

13. Composition pharmaceutique selon la revendication 11 pour administration locale, caractérisée en ce qu'elle comprend de 0,1% à 2% en poids d'agent actif par rapport à la composition totale.

14. Composition cosmétique contenant à titre d'agent actif au moins l'une des pyrido[3,4-c]furo[3,2-g]coumarines définies dans les revendications 1 à 4 en association avec un véhicule convenant à l'usage externe.

15. Composition cosmétique selon la revendication 14, caractérisée en ce qu'elle contient de 0,01 à 0,5% en poids d'agent actif.

**Revendications pour l'état contractant: AT**

1. Procédé pour l'obtention des pyrido[3,4-c]furo[3,2-g]coumarines de formule I:

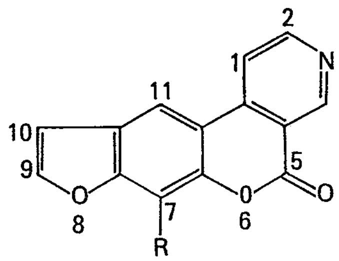

(I)

dans laquelle R représente un atome d'hydrogène, un groupe alkyle inférieur en $C_1$-$C_4$ ou un groupe alkoxy inférieur en $C_1$-$C_4$, caractérisé en ce que l'on fait réagir un acétate d'hydroxy-6 R-7 dihydro-2,3 benzofuranne de formule II:

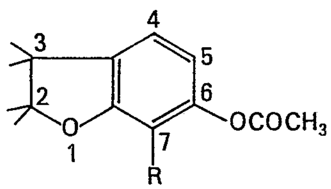

(II)

dans laquelle R est comme défini à la revendication 1, avec une carbéthoxy-3 pipéridone-4 de formule III:

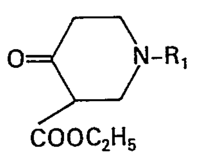

(III)

dans laquelle $R_1$ représente un atome d'hydrogène ou un substituant, ce qui conduit à une hexahydro-1,2,3,4,9,10 $R_1$-3 R-7 pyrido[3,4-c]furo[3,2-g]coumarine de formule IV:

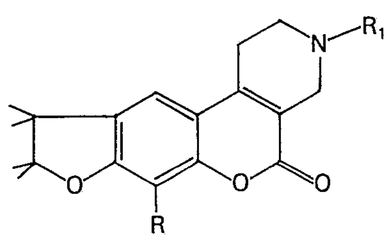

(IV)

dans laquelle R et $R_1$ sont définis comme précédemment, et que l'on soumet cette dernière à une réaction d'aromatisation au cours de laquelle on réalise à la fois la déshydrogénation et l'enlèvement d'un substituant $R_1$.

2. Procédé selon la revendication 1, caractérisé en ce que R est un groupe méthyle.

3. Procédé selon la revendication 1, caractérisé en ce que R est un groupe méthoxy.

4. Procédé selon la revendication 1, caractérisé en ce que R est un atome d'hydrogène.

5. Procédé selon l'une quelconque des revendications précédentes, caractérisé en ce que $R_1$ est un groupe benzyle.

6. Procédé selon l'une quelconque des revendications précédentes, caractérisé en ce que la première étape est réalisée en milieu acide.

7. Procédé selon la revendication 6, caractérisé en ce qu'on travaille en présence d'acide acétique contenant du gaz chlorhydrique.

8. Procédé selon l'une quelconque des revendications précédentes, caractérisé en ce que la réaction d'aromatisation est effectuée dans un solvant choisi parmi le diphényléther et la décaline, en présence d'un catalyseur de déshydrogénation.

9. Procédé selon la revendication 8, caractérisé en ce que le catalyseur de déshydrogénation est du palladium sur charbon.

10. Procédé d'obtenion d'une composition pharmaceutique dont l'agent actif est une pyrido[3,4-c]furo[3,2-g]coumarine de formule I, telle que définie dans l'une des revendications 1 à 4, caractérisé en ce que l'on mélange ledit agent actif avec un véhicule pharmaceutiquement acceptable.

11. Procédé selon la revendication 10 pour préparer une composition pharmaceutique pour administration par voie orale, caractérisé en ce qu'on prépare des doses unitaires contenant une quantité correspondant à une administration de 0,5 à 2 mg/kg de poids corporel.

12. Procédé selon la revendication 10 pour préparer une composition pharmaceutique pour administration locale, caractérisé en ce que les quantités mélangées sont telles que l'on obtienne une composition contenant de 0,1% à 2% en poids d'agent actif.

13. Procédé pour préparer une composition cosmétique, caractérisé en ce que l'on mélange une pyrido[3,4-c]furo[3,2-g]coumarine de formule I, telle que définie dans l'une des revendications 1 à 4, avec un véhicule convenant à l'usage externe.

14. Procédé selon la revendication13, caractérisé en ce que la composition obtenue comprend de 0,01 à 0,5% en poids d'agent actif.

**Patentansprüche für die Vertragsstaaten: BE, CH, DE, GB, IT, LI, LU, NL, SE**

1. Pyrido[3,4-c]psoralene oder Pyrido[3,4-c]furo[3,2-g]cumarine der allgemeinen Formel I

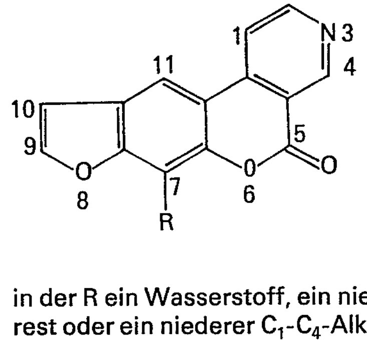

(I)

in der R ein Wasserstoff, ein niederer $C_1$-$C_4$-Alkylrest oder ein niederer $C_1$-$C_4$-Alkoxyrest ist.

2. Verbindung nach Anspruch 1 entsprechend der Formel I, in der R ein Methylrest ist.

3. Verbindung nach Anspruch 1 entsprechend der Formel I, in der R ein Methoxyrest ist.

4. Verbindung nach Anspruch 1 entsprechend der Formel I, in der R ein Wasserstoffatom ist.

5. Verfahren zur Herstellung von Pyrido[3,4-c]furo[3,2-g]-cumarinen der Formel I, dadurch gekennzeichnet, dass man ein 6-Hydroxy-7-R-2,3-dihydrobenzofuranacetat der Formel II

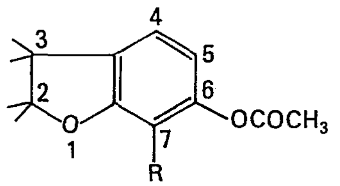

(II)

in der R die in Anspruch 1 definierten Bedeutungen hat, mit einem 3-Carbethoxy-4-piperidon der Formel III

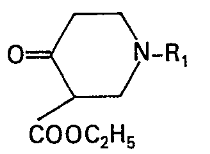

(III)

in der $R_1$ ein Wasserstoffatom oder ein Substituent ist, umsetzt, was zu einem 1,2,3,4,9,10-hexahydro-3-$R_1$-7-R-pyrido[3,4-c]furo[3,2-g]cumarin der Formel IV:

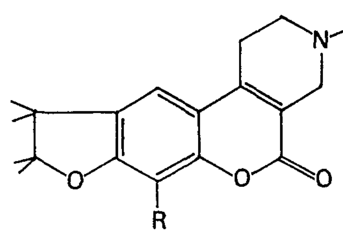

(IV)

in der R and $R_1$ die vorstehend genannten Bedeutungen haben, führt und die letztere einer Aromatisierungsreaktion unterwirft, in deren Verlauf man gleichzeitig die Dehydrierung und Entfernung eines Substituenten $R_1$ bewirkt.

6. Verfahren nach Anspruch 5, dadurch gekennzeichnet, dass $R_1$ eine Benzylgruppe ist.

7. Verfahren nach einem der Ansprüche 5 oder 6, dadurch gekennzeichnet, dass die erste Stufe in einem sauren Medium durchgeführt wird.

8. Verfahren nach Anspruch 7, dadurch gekennzeichnet, dass man in Gegenwart von Salzsäuregas enthaltender Essigsäure arbeitet.

9. Verfahren nach irgendeinem der Ansprüche 5 bis 8, dadurch gekennzeichnet, dass die Aromatisierungsreaktion in einem aus Diphenylether und Decalin ausgewählten Lösungsmittel in Gegenwart eines Dehydrierungskatalysators durchgeführt wird.

10. Verfahren nach Anspruch 9, dadurch gekennzeichnet, dass der Dehydrierungskatalysator Palladium auf Kohle ist.

11. Pharmazeutische Zubereitung, enthaltend als Wirkstoff wenigstens eines der in den Ansprüchen 1 bis 4 definierten Pyrido[3,4-c]furo[3,2-g]cumarine in Verbindung mit einem pharmazeutisch unbedenklichen Träger.

12. Pharmazeutische Zubereitung nach Anspruch 11 für die orale Verabreichung, dadurch gekennzeichnet, dass sie eine genügende Menge enthält, damit die verabreichte Einheitsdosis 0,5 bis 2 mg/kg Körpergewicht enthält.

13. Pharmazeutische Zubereitung nach Anspruch 11 für die örtliche Anwendung, dadurch gekennzeichnet, dass sie 0,1 bis 2 Gew.% Wirkstoff, bezogen auf die gesamte Zubereitung, enthält.

14. Kosmetische Zubereitung, enthaltend als Wirkstoff wenigstens eines der in den Ansprüchen 1 bis 4 definierten Pyrido[3,4-c]furo[3,2-g]cumarine in Verbindung mit einem für die äussere Anwendung geeigneten Träger.

15. Kosmetische Zubereitung nach Anspruch 14, dadurch gekennzeichnet, dass sie 0,01 bis 0,5 Gew.% Wirkstoff enthält.

**Patentansprüche für den Vertragsstaat: AT**

1. Verfahren zur Herstellung von Pyrido[3,4-c]furo[3,2-g]cumarinen der Formel I

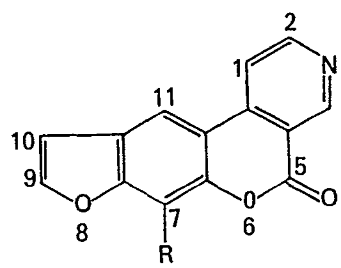

(I)

worin R ein Wasserstoffatom, eine niedere $C_1$-$C_4$-Alkylgruppe oder eine niedere $C_1$-$C_4$-Alkoxygruppe ist, dadurch gekennzeichnet, dass man ein 6-Hydroxy-7-R-2,3-dihydrobenzofuranacetat der Formel II

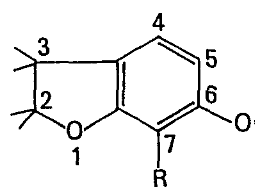

(II)

worin R die vorstehend genannten Bedeutungen hat, mit einem 3-Carbethoxy-4-piperidon der Formel III

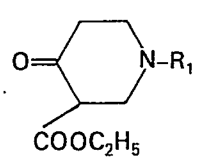

(III)

in der $R_1$ ein Wasserstoffatom oder ein Substituent ist, umsetzt, was zu einem 1,2,3,4,9,10-Hexa-

hydro-3-$R_1$-7-R-pyrido[3,4-c]furo[3,2-g]cumarin der Formel IV führt:

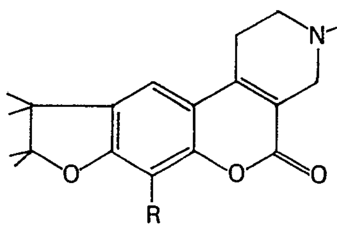

(IV)

worin R und $R_1$ die vorstehend genannten Bedeutungen haben, und dass man das letztere einer Aromatisierungsreaktion unterwirft, in deren Verlauf man gleichzeitig die Dehydrierung und die Entfernung eines Substituenten $R_1$ bewirkt.

2. Verfahren nach Anspruch 1, dadurch gekennzeichnet, dass R ein Methylrest ist.

3. Verfahren nach Anspruch 1, dadurch gekennzeichnet, dass R ein Methoxyrest ist.

4. Verfahren nach Anspruch 1, dadurch gekennzeichnet, dass R ein Wasserstoffatom ist.

5. Verfahren nach irgendeinem der vorstehenden Ansprüche, dadurch gekennzeichnet, dass $R_1$ eine Benzylgruppe ist.

6. Verfahren nach irgendeinem der vorstehenden Ansprüche, dadurch gekennzeichnet, dass die erste Stufe in einem sauren Medium durchgeführt wird.

7. Verfahren nach Anspruch 6, dadurch gekennzeichnet, dass man in Gegenwart von Salzsäuregas enthaltender Essigsäure arbeitet.

8. Verfahren nach irgendeinem der vorstehenden Ansprüche, dadurch gekennzeichnet, dass die Aromatisierungsreaktion in einem aus Diphenylether und Decalin ausgewählten Lösungsmittel in Gegenwart eines Dehydrierungskatalysators durchgeführt wird.

9. Verfahren nach Anspruch 8, dadurch gekennzeichnet, dass der Dehydrierungskatalysator Palladium auf Kohle ist.

10. Verfahren zur Herstellung einer pharmazeutischen Zubereitung, deren Wirkstoff ein Pyrido[3,4-c]furo[3,2-g]cumarin der Formel I ist, wie sie in einem der Ansprüche 1 bis 4 definiert wurde, dadurch gekennzeichnet, dass man den Wirkstoff mit einem pharmazeutisch unbedenklichen Träger mischt.

11. Verfahren nach Anspruch 10 zur Herstellung einer pharmazeutischen Zubereitung für die orale Verabreichung, dadurch gekennzeichnet, dass man Einheitsdosen herstellt, die einer Verabreichung von 0,5 bis 2 mg/kg Körpergewicht entsprechen.

12. Verfahren nach Anspruch 10 zur Herstellung einer pharmazeutischen Zubereitung für die örtliche Anwendung, dadurch gekennzeichnet, dass die gemischten Mengen derart sind, dass man eine Zubereitung erhält, die 0,1 bis 2 Gew.% Wirkstoff enthält.

13. Verfahren zur Herstellung einer kosmetischen Zubereitung, dadurch gekennzeichnet, dass man ein Pyrido[3,4-c]furo[3,2-g]cumarin der Formel I, wie sie in einem der Ansprüche 1 bis 4 definiert wurde, mit einem für die äussere Anwendung geeigneten Träger mischt.

14. Verfahren nach Anspruch 13, dadurch gekennzeichnet, dass die erhaltene Zubereitung 0,01 bis 0,5 Gew.% Wirkstoff enthält.

**Claims for the Contracting States: BE, CH, DE, GB, IT, LI, LU, NL, SE**

1. Pyrido[3,4-c]psoralenes or pyrido[3,4-c]furo[3,2-g]coumarins corresponding to the general formula I:

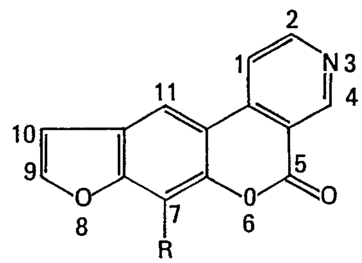

(I)

in which formula R denotes a hydrogen atom, a $C_1$-$C_4$-lower alkyl group or a $C_1$-$C_4$ lower alkoxy group.

2. Compound according to Claim 1, corresponding to formula I in which R is a methyl group.

3. Compound according to Claim 1, corresponding to formula I in which R is a methoxy group.

4. Compound according to Claim 1, corresponding to formula I in which R is a hydrogen atom.

5. Process for the preparation of pyrido[3,4-c]furo[3,2-g]coumarins of formula I, characterized in that an acetate of 6-hydroxy-7-R-2,3-dihydrobenzofuran of formula II:

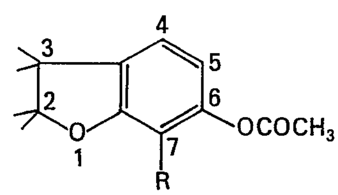

(II)

in which R is a defined in Claim 1, is reacted with a 3-carbethoxy-4-piperidone of formula III:

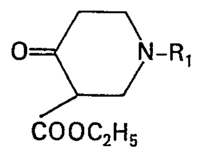

(III)

in which $R_1$ denotes a hydrogen atom or a substituent, which yields a 1,2,3,4,9,10-hexahydro-3-$R_1$-7-R-pyrido[3,4-c]furo[3,2-g]coumarin of formula IV:

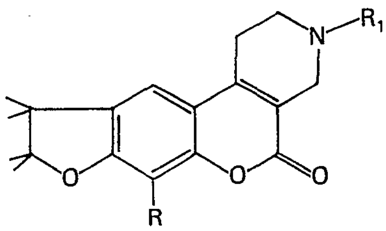

(IV)

in which R and $R_1$ are defined as above, and that the latter is subjected to an aromatization reaction during which both the dehydrogenation and the removal of a substituent $R_1$ are carried out.

6. Process according to Claim 5, characterized in that $R_1$ is a benzyl group.

7. Process according to either of Claims 5 and 6, characterized in that the first stage is carried out in an acid medium.

8. Process according to Claim 7, characterized in that it is carried out in the presence of acetic acid containing hydrogen chloride gas.

9. Process according to any one of Claims 5 to 8, characterized in that the aromatization reaction is carried out in a solvent chosen from diphenyl ether and decalin, in the presence of a dehydrogenation catalyst.

10. Process according to Claim 9, characterized in that the dehydrogenation catalyst is palladium on charcoal.

11. Pharmaceutical composition containing as an active agent at least one of the pyrido[3,4-c]furo[3,2-g]coumarins defined in Claims 1 to 4, in combination with a pharmaceutical acceptable carrier.

12. Pharmaceutical composition according to Claim 11 for oral administration, characterized in that it comprises a sufficient quantity for the unit dose administered to be 0.5 to 2 mg/kg of body weight.

13. Pharmaceutical composition according to Claim 11 for local administration, characterized in that it comprises from 0.1% to 2% by weight of active agent based on the total composition.

14. Cosmetic composition containing as an active agent at least one of the pyrido[3,4-c]furo[3,2-g]coumarins defined in Claims 1 to 4, in combination with an appropriate carrier for external use.

15. Cosmetic composition according to Claim 14, characterized in that it contains from 0.01 to 0.5% by weight of active agent.

**Claims for the Contracting State: AT**

1. Process for the preparation of pyrido[3,4-c]furo[3,2-g]coumarins of formula 1:

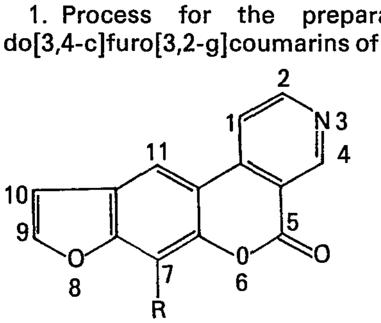

(I)

in which R denotes a hydrogen atom, a $C_1$-$C_4$ lower alkyl group or a $C_1$-$C_4$ lower alkoxy group, characterized in that an acetate of 6-hydroxy-7-R-2,3-dihydrobenzofuran, of formula II:

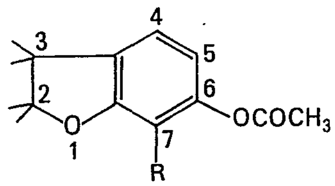

(II)

in which R is as defined in Claim 1, is reacted with a 3-carbethoxy-4-piperidone of formula III:

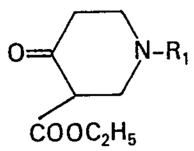

(III)

in which $R_1$ denotes a hydrogen atom or a substituent, which yields a 1,2,3,4,9,10-hexahydro-3-$R_1$-7-R-pyrido[3,4-c]furo[3,2-g]coumarin of formula IV:

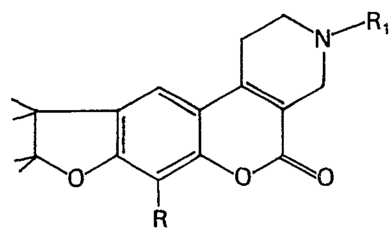

(IV)

in which R and $R_1$ are defined as above, and that the latter is subjected to an aromatization reaction during which both the dehydrogenation and the removal of a substituent $R_1$ are carried out.

2. Process according to Claim 1, characterized in that R is a methyl group.

3. Process according to Claim 1, characterized in that R is a methoxy group.

4. Process according to Claim 1, characterized in that R is a hydrogen atom.

5. Process according to any one of the preceding claims, characterized in that $R_1$ is a benzyl group.

6. Process according to any one of the preceding claims, characterised in that the first stage is effected in an acid medium.

7. Process according to Claim 6, characterized in that it is carried out in the presence of acetic acid containing hydrogen chloride gas.

8. Process according to any one of the preceding claims, characterized in that the aromatization reaction is carried out in a solvent chosen from diphenyl ether and decalin, in the presence of a dehydrogenation catalyst.

9. Process according to Claim 8, characterized in that the dehydrogenation catalyst is palladium on charcoal.

10. Process for the preparation of a pharmaceutical composition in which the active agent is a pyrido[3,4-c]furo[3,2-g]coumarin of formula I,

as defined in one of Claims 1 to 4, characterized in that the said active agent is mixed with a pharmaceutically acceptable medium.

11. Process according to Claim 10, for preparing a pharmaceutical composition for oral administration, characterized in that unit doses containing a quantity corresponding to an administration of 0.5 to 2 mg/kg of body weight are prepared.

12. Process according to Claim 10, for preparing a pharmaceutical composition for local administration, characterized in that the mixed quantities are such that a composition containing from 0.1% to 2% by weight of active agent is obtained.

13. Process for preparing a cosmetic composition, characterized in that a pyrido[3,4-c]furo[3,2-g]coumarin of formula I, as defined in one of Claims 1 to 4, is mixed with an appropriate carrier for external use.

14. Process according to Claim 13, characterized in that the composition obtained comprises from 0.01 to 0.5% by weight of active agent.